# EUROPEAN PATENT APPLICATION

(11) **EP 0 903 341 A1**
(43) Date of publication of application: **24.03.1999**
(21) Application number: 98202385.5
(22) Date of filing: 16.07.1998
(51) Int. Cl.: C07C 323/41, C07C 323/36, A61K 31/16, A61K 31/135

(54) **"Novel ortho-mercaptoaniline compounds"**

(30) Priority: 22.07.1997 EP 97202283
(71) Applicant: Akzo Nobel N.V., 6824 BM Arnhem (NL)
(72) Inventor: Chaudhuri, S.K., 700016 Calcutta (IN); Poisson, C., 63200 Riom (FR)
(74) Representative: Kraak, Hajo

(57) **Abstract**

An ortho-mercaptoaniline derivative having the general formula 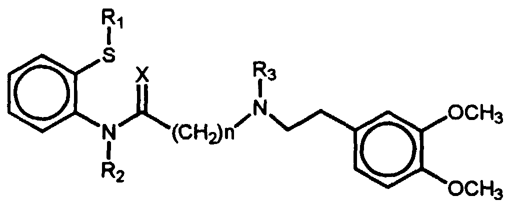 wherein X is (H,H) or O; n is 1 or 2; and
R₁ is (C₁-C₆)alkyl or phenyl-(C₁-C₆)alkylene, optionally substituted at phenyl with a (C₁-C₆)alkyloxy group;
R₂ is (C₁-C₆)alkyl or (C₁-C₆)alkyloxymethyl;
R₃ is hydrogen or methyl, with the proviso that R₃ is hydrogen if R₁ is methyl;
and acid addition salts thereof;
a process for the preparation of such a compound, and a pharmaceutical composition comprising such a compound for anti-amoebal action or the treatment of dysentery.

## Description

This invention relates to novel ortho-mercaptoaniline compounds, to a process for the preparation thereof and to the use of these compounds for the preparation of medicaments for the treatment of amoebal infections such as dysentery.

Almost 2 billion cases of diarrhoea infections per year occur in the world, 3 million of these cases lead to death of the affected person. Most of the fatalities fall among children with nutritional deficiencies. A survey in India resulted in an estimate that 20% of deaths due to diarrhoea is due to dysentery. Infection by the trophozoites Entamoeba histolytica is one of the most common causes of dysentery. There is no drug available which can reliably kill both the trophic and the cystic stages of Entamoeba histolytica in the lumen of large intestine and also kill the trophozoites invading the wall of intestine, liver and other organs. Treatment with more effective and better tolerated medicaments is important in the control of this infectious disease. Metronidazole, ornidazole and tinidazole are available for treatment of dysentery due to Entamoeba histolytica, but these compounds induce side effects. Side effects occur with these drugs in 5-60 % of treated patients according to different reports which vary in their criteria for severity of the side effect. Among those side effects are vertigo, nausea and a severely disturbed taste. A warning for carcinogenicity of metronidazole is issued in reference texts.
The present invention provides ortho-mercaptoanilinine compounds having the structural formula I, 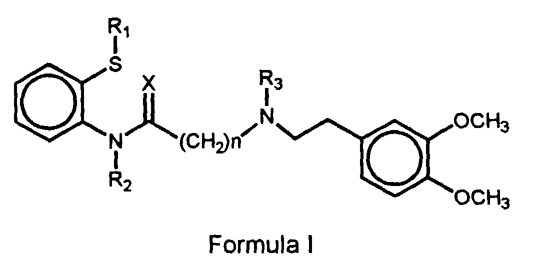 wherein
X is (H,H) or O; n is 1 or 2; and
R₁ is (C₁-C₆)alkyl or phenyl-(C₁-C₆)alkylene, optionally substituted at phenyl with a (C₁-C₆)alkyloxy group;
R₂ is (C₁-C₆)alkyl or (C₁-C₆)alkyloxymethyl;
R₃ is hydrogen or methyl with the proviso that R₃ is hydrogen if R₁ is methyl;
and acid addition salts thereof.
The indication "(C₁-C₆)alkyl" is used here to represent a straight or branched aliphatic alkyl group such as methyl, ethyl, propyl, isopropyl, butyl, isobutyl, t-butyl, amyl, isoamyl, hexyl and the like. Methyl is preferred.
The indication "(C₁-C₆) alkylene" is used here to represent a bivalent branched or unbranched alkyl radical having 1 - 6 carbon atoms such as methylene, 1,2 ethane-di-yl, 1,3 propane-di-yl and the like. Methylene is preferred.
The compounds of formula I have anti-amoebal activity and are useful for the treatment of dysentery. These compounds do not have a disturbing effect on taste in man.
Preferred compounds of formula I according to the invention are the compounds 3-[2-(3,4-dimethoxyphenyl)ethylamino]-N-methyl-N-[(2-methylthio)phenyl]-propionamide (Compound A) 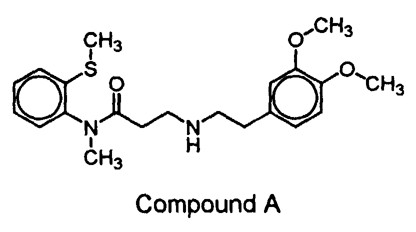 and acid addition salts thereof,
and N-[2-(3,4-dimethoxyphenyl)ethyl]-N-methyl-N'-[2-(4-methoxyphenyl)methylthiophenyl]-N'-methyl-1,3-diaminopropane (Compound B) 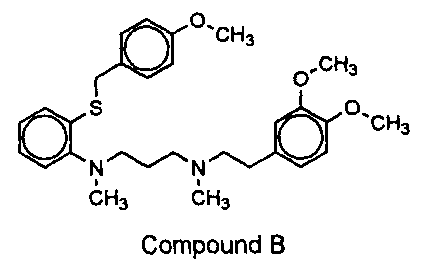 and acid addition salts thereof.

Compounds of this invention in which X is oxygen may be prepared by a condensation reaction between suitable N-(2-halo-acetyl)- or N-(3-halo-propionyl)thioanilines with homoveratrylamine or N-methyl-(homoveratryl)amine as illustrated in the preparation scheme below (step d). The terms halo- and halogen are used to indicate F, Cl, Br, or I. Cl is preferred. Compounds of formula I in which X is two hydrogen atoms, may be obtained by means of a suitable reduction, for example with lithiumaluminiumhydride or boronhydride, of the corresponding acetamide or propionamide (Scheme step e). 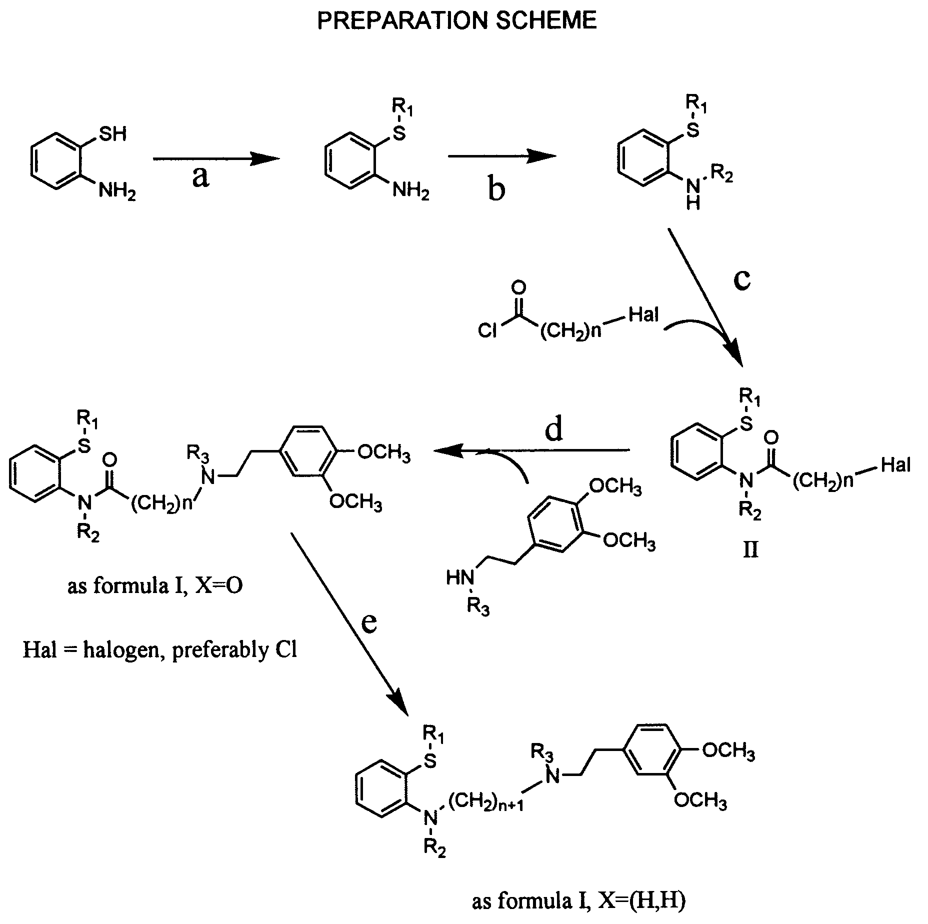 The 2-halo-acetamides and the 3-halo-propionamides according to formula II in the Preparation Scheme, may be prepared by reacting the suitably substituted mercaptoaniline with 3-halo-propionylchloride or halo-acetylchloride (scheme step c). Thioanilines of which the sulfhydryl hydrogen is substituted by a suitable alkyl or aryl function may be (ar)alkylated on the amino group, for instance by reductive (ar)alkylation methods (preparation scheme, step b). More specifically, N-methylation can be carried out by reacting the thiosubstituted thioaniline with ethyl chloroformate followed by reduction of the carbonyl group with a reducing reagent such as lithiumaluminiumhydride or boronhydride.
A suitable S-alkyl- or S-aryl-thioaniline is made in a process in which 2-aminothiophenol as starting material is alkylated or arylated on the sulphur atom by a suitable alkyl halide or aryl halide (chloride is the preferred halogen), preferably in a biphasic reaction medium. Such biphasic medium may be a mixture of toluene and water. The phase-transfer catalyst Adogen 464R (methyltrialkyl (C₈-C₁₀) ammonium chloride) can be used to optimise the process.

The anti-amoebal action of these compounds can for example be demonstrated in an in vitro test with cultivated Entamoeba histolytica or on the cure index of rats infected with Entamoeba histolytica. The compounds of the invention can be used as free base or as acid addition salt. The acid addition salts are preferably the pharmaceutically acceptable, non-toxic addition salts with suitable acids, such as those of inorganic acids, for example hydrochloric, hydrobromic, nitric, sulphuric or phosphoric acids, or of organic acids, such as organic carboxylic acids, for example glycollic, maleic, fumaric, tartaric, citric, lactic or acetic acid.

The compounds of this invention can be incorporated in pharmaceutical formulations known to be suitable for enteral or parenteral administration to an infected person or animal. Accordingly the invention includes a pharmaceutical composition comprising as active ingredient a compound of formula I or a pharmaceutically acceptable acid addition salt thereof, associated with a pharmaceutically acceptable carrier. In making the compositions of the invention conventional techniques for the preparation of pharmaceutical compositions may be used. For example, a compound of formula I as the active ingredient will usually be mixed with a carrier, or diluted with a carrier, or enclosed within a carrier. When the carrier serves as a diluent, it may be solid, semi-solid or liquid material which acts as a vehicle, excipient or medium for the active ingredient. Some examples of suitable carriers are various carbohydrates, gum acacia, calcium phosphate, gelatin, talc, magnesium stearate or mineral oil. The compositions of the invention may, if desired, be formulated so as to provide quick, sustained or delayed release of the active ingredient after administration to an animal or patient.
Depending on the method of administration, the compositions may be formulated as tablets, capsules, injection solutions for parenteral use, suspensions or elixirs for oral use or suppositories for rectal use. Preferably the compositions are formulated in a dosage unit form, each containing active ingredient suitable for administration of an amount of 0.01 - 30 mg, more usually 0.1 - 15 mg, per kg body weight of the treated person or animal.
For treatment or prevention of amoebal infections and dysentery the exact dose and regimen of administration of the active ingredient will vary with the particular compound, the route of administration, and the age, size and condition of the indvidual subject or animal to whom the medicament is to be administered. A dosage for humans should preferably be within the range of 0.05 - 5 gram per person per day.

The invention is further explained by reference to the following examples:

### Example I

### Ia Method to prepare N-[2-methylthio)phenyl-carbaminc acid ethyl ester

To an ice-cold solution of 2-(methylthio)aniline (100 g, 0.72 mol) in water (2 l) and chopped ice (400-500 g), ethyl chloroformate (42 ml, 0.43 mol) was added dropwise with vigorous stirring and external cooling to maintain the temperature between 5-10°C. A solution of sodium hydroxide (34.4 g) in water (390 ml) was then added dropwise simultaneously with a second portion of ethyl chloroformate (42 ml, 0.43 mol). The mixture was stirred for an additional 2 h at 5-10°C. The reaction mixture was extracted with ether and the organic extract was washed with water and dried. Evaporation of solvent gave 152 g of N-(2-methylthio)phenyl-carbamic acid ethyl ester. GLC purity 99%; IR √C=O: 1740 cm⁻¹.

### Ib. Method to prepare N-methyl-2-(methylthio)aniline:

To a suspension of LiAlH₄ (26.8 g, 0.7 mol) in dry ether (800 ml) a solution of N-(2-methylthio)phenyl-carbamic acid ethyl ester (79 g, 0.35 mol) in dry ether (200 ml) was added dropwise at a rate which produced controlled reflux. The mixture was refluxed for 5 hrs. after addition. The progress of the reaction was monitored with IR spectroscopy.
The resulting mixture was cautiously hydrolysed with dropwise addition of water and filtered off. The ether portion was dried and concentrated under vacuum to give 50.7 g of a colourless liquid

### Ic. A method of making 3-chloro-N-methyl-(2-methylthio)phenyl-propionamide

To an ice-cold solution of N-methyl-2-(methylthio)aniline (98g, 0.64 mole) in 1 liter toluene were added pyridine (86 ml, 1.06 mole) and 3-chloropropionyl chloride (81.3 ml, 0.85 mole) simultaneously with stifling at a rate to keep the reaction temperature at 6°-10°C. Then the reaction mixture was allowed to come to room temperature and was then filtered and washed with water. The organic layer was dried and evaporated to give 155 g of the desired compound. The compound is a colourless liquid. GLC: 98%

### Id. Method of making 3-[2-(3,4-dimethoxyphenyl)-ethylamino]-N-methyl-N-[(2-methylthio)phenyl]-propionamide (E)-2-butenedioate (1:1)

A mixture of 3-chloro-N-methyl-(2-methylthio)phenyl-propionamide (70 g, 0.28 mole), homoveratrylamine (57.2 g, 0.32 moles) and potassium carbonate (79.2 g, .057 moles) in 700 ml n-butanol was refluxed for 8 hrs. Then the reaction mixture was filtered and the solvent was evaporated off. The residue was diluted with water and was extracted with methylene chloride. The organic layer was washed with water, dried and evaporated to give 104 g crude base which was converted in ethanol to the fumarate salt. Two crystallisations from ethanol, followed by crystallisation from ethanol-isopropanol-water (50:50:3 by volume) gave 90 g of the desired compound as a white crystalline powder. Melting point: 165 °C. GLC purity: 94 % (base); TLC : n-butanol/acetic acid/water (upper layer)(4:1:5 by volume): Rf: 0.45

### Example II

### IIa Method of making 4-methoxy benzylchloride

4-methoxy benzyl chloride is unstable and therefore not readily available. A convenient preparation from 4-methoxy benzyl alcohol is described here: To 4-methoxybenzyl alcohol (13.8 g; 12.5 ml; 0.1 mol), was added concentrated hydrochloride acid (24.8 ml). After stirring vigorously for 15 min the contents of the flask were transferred to a separatory funnel. The lower layer (4-methoxy benzyl chloride) was separated, dried over granular calcium chloride (2 g) for about 30 min, and filtered to remove the drying agent. 15.1 g of 4-methoxy benzylchloride was obtained. The compound is highly irritant, can be used only a few days after it is made, and should be stored in a cold, dry place.

### IIb. Method of making 2-(4-methoxy-phenylmethylthio)-aniline and 2-[(4-methoxy)phenylmethylthio]-N-methyl-aniline

2-aminothiophenol (130.5 g, 1.04 mol), NaOH (70 g), water (700 ml), methyltrialkyl (C₈-C₁₀) ammonium chloride (39 g), toluene (650 ml) and 4-methoxybenzyl chloride (170 ml, 1.25 mol) are vigorously stirred at 80°C for 5.5 hours. The reaction mixture is extracted with diethyl ether. The organic solution is washed with water, dried and evaporated. The solid residue (329 g) is purified by vacuum distillation (bp 160°C, 0.05 mmHg) and crystallised from isopropyl ether to give 222 g 2-(4-methoxy-phenylmethylthio)-aniline. The compound is a white solid (mp: 67.7°C TLC: n-butanol/water/acetic acid upper layer (4:5:1 by volume) Rf 0.95).
In order to obtain 2-(4-methoxy-phenylmethylthio)-N-methyl-aniline the compound 2-(4-methoxy-phenylmethylthio)-aniline is N-methylated according to the methods described in examples Ia and Ib above.

### IIc. Method of making 3-chloro-N-methyl-N-[(2-(4-methoxy)-phenylmethylthio)-phenyl]-propionamide

3-Chloropropionylchloride (15 ml, 0.15 mol) was added dropwise to a stirred solution of 2-[(4-methoxy)-phenylmethylthio]-N-methyl-aniline (33 g, 0.13 mol) in toluene (330 ml) and pyridine (30 ml, 0.38 mol) at 10°C. The mixture was allowed to come to room temperature and filtered. The filtrate was diluted with ether, washed with water, dried, and evaporated to give 43 g of 3-chloro-N-methyl-N-[(2-(4-methoxy)-phenylmethylthio)-phenyl]-propionamide. IR: √C=O: 1668 cm⁻¹.

### IId. Method of making 3-[(2-(3,4-dimethoxyphenyl)ethyl)amino]-N-methyl-N-[(2-(4-methoxy)-phenylmethylthio)-phenyl]-propionamide

A mixture of 3-chloro-N-methyl-N-[(2-(4-methoxy)-phenylmethylthio)-phenyl]-propionamide (30 g, 0.086 mol), homoveratrylamine (15 ml, 0.086 mol) and potassium carbonate (35 g, 0.25 mol) in n-butanol (300 ml) was refluxed for 8 h. The progress of the reaction was monitored with TLC. The reaction mixture was filtered, and butanol was evaporated. The residue was diluted with water and extracted with ether. The organic layer was washed with water, dried and evaporated to give 24.6 g of the desired compound. The crude base was purified by flash chromatography on silica gel (300g) with hexane-ethanol-isopropanol-ammonia (80:10:10:0.5 then 50:25:25:1 by volume) to give two batches of which the second one (12.3 g) was 98.5 % pure compound.

### IIe Method of making N-methyl-N-[(2-(4-methoxy)phenylmethylthio)-phenyl]-N'-[2-(3,4-dimethoxyphenyl)ethyl] diaminopropane

To a stirred suspension of NaBH₄ (4.4 g, 0.12 mol) in THF (20 ml), was added dropwise under N₂ at 0°C a solution of 3-[(2-(3,4-dimethoxyphenyl)-ethyl)amino]-N-methyl-N-[(2-(4-methoxy)-phenylmethylthio)-phenyl]-propionamide (22 g, 0.044 mol) in tetrahydrofuran (THF) (200 ml) and then dropwise and very slowly (∼2h) a solution of boron trifluoride diethyl etherate (32 ml) in THF (32 ml). The reaction mixture was allowed to come to room temperature and was then stirred for 4 hrs. The progress of the reduction was monitored by infrared spectroscopy. The mixture was treated at 0°C with 10% HCl (100 ml), and THF was distilled off at atmospheric pressure. The aqueous phase was basified at 0°C and extracted with dichloromethane. The organic solution was washed, dried and evaporated to give 20.5 g of 84% pure (GLC) N-methyl-N-[(2-(4-methoxyphenyl)-methylthio)-phenyl]-N'-[2-(3,4-dimethoxyphenyl)ethyl] diaminopropane.

Some of the crude base was converted to the hemifumarate in isopropyl ether/ethanol. Three recrystallisations from ethanol gave the hemifumarate salt. This compound is a white solid (mp:151.2 °C; HPLC purity: 99.2%; TLC: chloroform/methanol/acetic acid (5:1:0.3 by volume); Rf: 0.64.

### IIf. Method of making N-[2-(3,4 dimethoxyphenyl)-ethyl]-N-methyl-N'-[(2-(4-methoxy)phenylmethylthio)-phenyl]-N'-methyl-1,3 diaminopropane

To a stirred solution of N-methyl-N-[(2-(4-methoxy)phenylmethylthio)-phenyl]-N'-[2-(3,4-dimethoxyphenyl)ethyl] diaminopropane (9.3 g, 0.019 mol) in acetonitrile (50 ml), was added dropwise formaldehyde (9 g, 0.09 mol, 36.5% aqueous solution), and portionwise sodium cyanoborohydride (1.5 g, 0.023 mol) keeping the reaction at room temperature. The mixture was stirred at room temperature for 2 h and acidified to pH 3-4 with acetic acid. The mixture was stirred again at room temperature for 1.5 h. Acetonitrile was removed in vacuo. The residue was basified with 10 N sodium hydroxide solution and extracted with ether. The organic layer was dried and concentrated in vacuo. From the residue (9.7 g) the desired compound was isolated by flash chromatography on silica gel (110 g) with hexane/ isopropanol/ ethanol/concentrated ammonia (80:10:10:0.1 by volume) as eluant to give 2.1 g of 98 % pure N-[2-(3,4 dimethoxyphenyl)-ethyl]-N-methyl-N'-[2-(4-methoxyphenylmethylthiophenyl]-N'-methyl-1,3 diaminopropane.
The crude base was converted to the sesquifumarate in diethyl ether. Washing from diethyl ether gave the desired sesquifumarate salt. The compound is a white solid (mp: 100.2°C; HPLC purity: 99.1%; TLC: chloroform/methanol/acetic acid (5:1:0.3): Rf: 0.67.

### Example III

### Demonstration of anti-amoebic action in vitro

Axenic Entamoeba histolytica cultures were grown in glass, screw-capped tubes (16x100 mm) containing about 10 ml complete growth medium TYI-S-33. The tubes were inoculated with 0.5 ml of a 72 h old axenic culture of Entamoeba histolytica 200: NIH or E-32, obtained from an external supplier (Kothari Research Centre, Calcutta, India). After incubation for 24 h at 37°C compounds to be tested were added at desired concentrations. Incubation of the tubes was continued for an additional 48 h. The number of amoebae in the medium were counted by microscopic examination at 100x magnification.
The complete growth medium TYI-S-33 medium was prepared by mixing aseptically 9 ml of a nutrient solution, 1 ml of animal serum, 0.3 ml of a vitamin mixture and 0.05 ml of a solution containing the antibiotics streptomycine and benzylpenicillin. The composition of TYI-S-33 medium and method of preparation is described in detail by Diamond (J Parasitology 54:1047-1056, 1968).
Test compounds are tested at different concentrations, the highest is 100 µg/ml. Lower concentrations are tested in successive tests until the minimum inhibitory concentration is reached. The reference compounds tinidazole was active in killing the amoeba at a minimum concentration of 1.5 µg/ml. Compound B of this invention was active in killing the amoeba at a minimum concentration of 3.1 µg/ml.

### Example IV

### Demonstration of anti-amoebic action in vivo in an infected animal.

Juvenile three weeks old male and female rats weighing 25-28 g were infected with Entamoeba histolytic under anaesthesia with ether. They were kept fasting during the night before surgery. For inoculation of the caecum the caecum was exposed via a right paramedian incision of the lower abdomen. The inoculum in a quantity of 0.2 ml containing 3.0 x 10⁵ Entamoeba histolytica is injected into the blind end of the caecum. The incision is sutured by wound clips and antiseptic powder containing neomycin, bacitracin and sulphonamide is applied to the wound. Availability of the usual diet is resumed 4 hrs after infection. In each experiment there were control groups treated with injection vehicle or reference compound and groups receiving a dose of a compound described in this invention. The group size per experiment was 5-7 animals per group. The treatment was started two days after infection and continued for five days. The route of administration was oral. Eight days after infection the (surviving) animals were sacrificed. The consequences of infection was assessed by the number of animals surviving and macroscopic and microscopic examination of the caecum. To each animal a virulence index was assigned. When dying and/or the content of the caecum contained no solid matter and the wall was shapeless with extensive ulceration a maximum virulence score of 8 was assigned. When the content was slightly less solid than normal and the wall was slightly thicker than normal a virulence score of 2 was assigned. Between these extremes intermediate scores were assigned for intermediate degrees of pathology. The animal was considered to be cured (score 0) when there were no Entamoeba histolytica present even in a cultivated growth medium contacted with caecal tissue from an apparently cured animal. The average virulence score was divided by the average virulence score of the vehicle treated control group and expressed as percentage of the control group. This is the cure index. No increase in cure index was seen with doses of 9 or 20 mg/kg per day of compound A, but with doses above this the cure index was increased as shown in the following table:

**Table**

| Anti amoebal activity in infected rats | | |
|---|---|---|
| Compound | Dose (mg/kg) oral | cure index |
| Tinidazole | 75 | 51 ± 8 |
| | 150 | 66 ± 5 |
| | 200 | 81 ± 5 |
| Compound A | 9 | 23 |
| | 18.8 | 34 |
| | 37.5 | 56 ±5 |
| | 75 | 76 ±6 |
| | 100 | 74 ± 10 |

While the preferred embodiments of this invention have been described and illustrated, various substitutions and modifications may be made hereto without departing from the scope of the invention. Accordingly, it is to be understood that the present invention has been described by way of illustration and not limitation.

## Claims

1. An ortho-mercaptoaniline derivative having the general formula 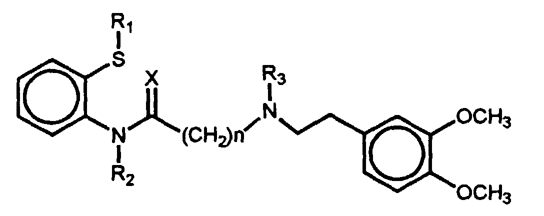 wherein X is (H,H) or O; n is 1 or 2; and
R₁ is (C₁-C₆)alkyl or phenyl-(C₁-C₆)alkylene, optionally substituted at phenyl with a (C₁-C₆)alkyloxy group;
R₂ is (C₁-C₆)alkyl or (C₁-C₆)alkyloxymethyl;
R₃ is hydrogen or methyl, with the proviso that R₃ is hydrogen if R₁ is methyl;
or acid addition salts thereof.

2. The ortho-mercaptoaniline derivative having the general formula of claim 1 wherein R₃ is hydrogen.

3. The ortho-mercaptoaniline derivative having the general formula of claim 1 wherein R₁ is a straight or branched alkyl with 2 to 6 carbon atoms or a phenyl-(C₁-C₆)alkylene, optionally substituted at phenyl with a (C₁-C₆)alkyloxy group; and R₃ is a methyl.

4. The ortho-mercaptoaniline derivative having the formula of claim 1 wherein X is O, R₁ is methyl, R₂ is methyl, R₃ is hydrogen and n=2.

5. The ortho-mercaptoaniline derivative having the formula of claim 1 wherein X is (H,H) and R₁ is 4-methoxyphenylmethyl; R₂ is methyl; R₃ is methyl and n=2.

6. A process for the preparation of the compounds of claim 1 characterised by: a condensation reaction of a compound of the formula 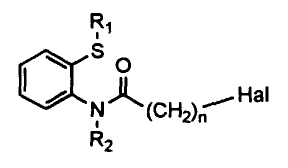
,in which n is 1 or 2; Hal is halogen; R₁ is (C₁-C₆)alkyl or phenyl-(C₁-C₆)alkylene, optionally substituted at phenyl with a (C₁-C₆)alkyloxy group; and R₂ is (C₁-C₆)alkyl or (C₁-C₆)alkyloxymethyl,
with homoveratrylamine or N-methyl-homoveratrylamine, optionally followed by reduction with a reducing agent and/or conversion to an acid additon salt.

7. A pharmaceutical composition comprising a compound according any one of the claims 1-5 in admixture with a pharmaceutically acceptable carrier.

8. The use of a compound according any one of the claims 1-5 for the manufacture of a composition for anti-amoebal action.

9. The use of a compound according any one of the claims 1-5 for the preparation of a medicament for the treatment of dysentery.
